# EUROPEAN PATENT SPECIFICATION

(11) **EP 1 267 635 B1**
(45) Date of publication and mention of the grant of the patent: **29.09.2010**
(21) Application number: 01917323.6
(22) Date of filing: 04.04.2001
(51) Int. Cl.: A23K 1/16, A23K 1/18, A61K 31/195, A61K 38/05

(54) **SUPPLEMENTATION OF EQUINE FEEDSTUFFS**
SUPPLEMENTIERUNG VON PFERDEFUTTER
APPORT COMPLEMENTAIRE DES ALIMENTS POUR EQUIDES

(30) Priority: 04.04.2000 GB 0008249
(43) Date of publication of application: 02.01.2003
(73) Proprietor: Mars, Incorporated, McLean, VA 22101 (US)
(72) Inventor: HARRIS, R. C. Waltham Centre for Pet Nutrition, Leicestershire LE14 4RT (GB); HARRIS, P. A. Waltham Centre for Pet Nutrition, Leicestershire LE14 4RT (GB)
(74) Representative: Cornish, Kristina Victoria Joy
(86) International application number: PCT/GB2001/001557
(87) International publication number: WO 2001/074173

(56) References cited:
- WO-A-97/33488
- WO-A-99/25331
- WO-A-99/65335
- FR-A- 2 758 243
- A. LINDNER: "Conference on equine sports medicine and science, 26-28 April, 1998 Cordoba, Spain" 1998 , WAGENINGEN PERS. , WAGENINGEN, NL XP001011542 & HARRIS PA ET AL.: "3. Nutritional ergogenic aids in the horse - uses and abuses, pages 203-218" page 211, paragraph 3

## Description

The present invention provides equine feedstuffs and methods by which plasma glutamine concentrations in equine animals can be increased or maintained.

Glutamine is a neutral amino acid which is readily transported across plasma membranes. As an important intermediate in a number of metabolic pathways, cellular utilisation of glutamine can far exceed that of other amino acids especially within intestinal and immune cells. Glutamine is important in the transport of amino nitrogen and ammonia, as a substrate in gluconeogenesis and ammoniagenesis, as a fuel source for rapidly dividing cells and may also be involved in the regulation of protein synthesis.

Under normal conditions, the body's requirement for glutamine is met by synthesis within specific tissues, principally skeletal muscle, and, to some extent, from dietary protein. Glutamine is, however, the preferential fuel source for intestinal enterocytes. The uptake of dietary glutamine by these cells is thought to be substantial, and that comparatively little is released into the bloodstream.

The high rate of glutamine utilisation by the intestine (identified to occur in man) may, in part, be due to the large lymphocyte and macrophage populations in intestinal walls and Peyer's patches. These cells exhibit high glutaminase activity and utilise glutamine as their preferential fuel source, even in the quiescent state. However, as with many other cells that require glutamine, both enterocytes and lymphocytes lack the synthetic apparatus to produce glutamine relying solely on circulatory or dietary sources. It is suggested that a fall in the plasma concentration could compromise lymphocyte function accounting for the increase in susceptibility to viral infection.

Infection and tissue injury will both draw upon the central glutamine pool reducing the plasma concentration. Recently, a 30% decline in the plasma concentrations in horses over the six days following exposure to viral challenge has been reported

FR-A-2 758 243 discloses nutritive compositions for race horses comprising amino acids and further additives such as glutamine.

The present invention provides means for increasing and maintaining plasma glutamine concentrations in equine animals. The equine animal may be clinically healthy or may be athletically or clinically stressed. Increasing glutamine consumption by increasing the level of a food substance rich in glutamine is not usually suitable, as this involves increasing the amount of protein ingested. Protein above required levels cannot be stored and is either used to produce energy - but this is a very inefficient process and results in a large amount of heat which the horse needs to get rid of --or the nitrogen has to be removed via urea. The potential disadvantages of excessive protein intakes (especially in the exercising animal) include:-
- increased utilisation of dietary protein for energy which increases the heat loan to the horse (as described above)
- increased urea production with concurrent increased urea levels in the blood and ammonia levels in the urine, and therefore the stable environment - which potentially may irritate the stabled animal's respiratory tract
- increased water requirements as needs to produce additional urine to excrete the urea (may be an additional adverse factor when dehydration is a possibility)
- above certain levels may exceed the capacity of the urea cycle - leading to metabolic problems
- above certain levels (not given) adversely affect glycogen storage.

Accordingly, a first aspect of the present invention relates to an equine feedstuff comprising a glutamine source of from 15 to 200mg/kg bodyweight per day free glutamine or an equivalent thereof, preferably 15 to 150mg/kg bodyweight, more preferably 15 to 100mg/kg bodyweight. Preferably, the glutamine is present at a level of from 30mg/kg bodyweight per day, most preferably at a level of up to 60mg/kg bodyweight per day, where the concentration given is for free glutamine or an equivalent level from an alternative glutamine source. Preferably the glutamine level above is a supplemented glutamine source (i.e. an added glutamine level to the glutamine already occurring in the raw materials of the feedstuff).

Equine animals of the present invention include the horse (in particular, working and racing horses), the donkey, the zebra and the camel.

The equine feedstuff is preferably composed of one or more of a hydrolysable carbohydrate source, one or more of a fibre source and one or more of a protein source, together with optional vitamins and minerals, molasses and oils (or fats). Hydrolysable carbohydrate sources include cereal sources such as oats, corn and barley. Fibre sources include grasses (preserved or other), sugar beet, soya hulls and oats. Protein sources include soya meal and linseed. Molasses is usually added as a palatability enhancer. The feedstuff may comprise simply cereals and oil or molasses, with the supplemented glutamine.

Preferably, the oil is of vegetable origin, preferably one or more highly polyunsaturated vegetable oil. Examples are corn oil, soya oil, or processed canola oil. Alternatively, the oil or fat can be added as a component containing oil or fat, such as oil-rich seeds or full fat soya. Other preferred fats are more saturated fats, which are more stable and thus less prone to rancidity. Examples are coconut oil, palm oil or sunflower oil. Oil may have the added benefit of reducing the insulin response to the diet so enabling further uptake of amino acids and may delay gastric emptying to a more prolonged steady trickle into the gut. The present invention provides a particular benefit when the oil is present in the feedstuff at a level of from 2 % or 4% by weight, preferably up to 20 % by weight, most preferably from 7% by weight, optionally up to 14 % by weight. A feedstuff with such an oil content results not only in an increase in the plasma glutamine level, but also an increase in the total time that the plasma glutamine level remains above the normal plasma glutamine baseline.

The glutamine source may be glutamine, such as a free amino acid L-glutamine or a peptide rich in L-glutamine or a glutamine rich extract. Suitable peptides rich in L-glutamine include dipeptides, tripeptides, tetrapeptides, pentapeptides, hexapeptides, longer chain peptides or peptide mixtures. Such peptide mixtures include proteins rich in L-glutamine, hydrolates or fractions thereof (e.g. gliadin, beans or lentils). Other L-glutamine derivatives include L-glutamine salts, N-acyl derivatives of L-glutamine including N-alkanoyl L-glutamine compounds such as N-acetyl L-glutamine. The N-acylation of L-glutamine stabilises the peptide, in comparison with free amino acid L-glutamine. Such stabilised peptide enables a longer shelf life for equine feedstuff according to the present invention. Such peptides may be pH and fluid stable. The dipeptide L-alanyl-L-glutamine is a particularly useful dipeptide. Typically a glutamine source of wheat protein hydrolate provides an equivalent of 30 % free glutamine. The extract can be obtained by conventional methods from a source containing glutamine (e.g. quadin, beans or lentils).

The formulation of the supplemented equine feedstuff is not limiting. Typically the supplement of glutamine source will be admixed together with the standard feedstuff used. The mixing may occur when the feedstuff is prepared or packaged or may occur when the feedstuff is provided to the animal. The supplementation may alternatively be supplied as a topping to the feedstuff. The feedstuff may also comprise a drink (for example, an aqueous or oil-based drink with the glutamine supplement) or be in the form of a snack bar. The drink, snack bar or other feedstuff may allow for easy transport, administration and monitoring of precise supplement given. When the feedstuff is in the form of a snack bar, the glutamine supplement may be admixed with the components or applied as a topping. The feedstuff may also be provided in pellet form.

In all cases the glutamine supplement may be in any form, such as in the form of a liquid or a solid, for example, a powder.

The complementary feedstuff of the present invention may comprise diets with the following analysis:

| Oil % | Protein % | Crude Fibre % | Ash % |
|---|---|---|---|
| Typical ranges: 2-20 | 8-16 | 2-20 | 5-20 |
| Examples: 14.5 | 13.5 | 4.20 | 6.5 |
| 8.0 | 12.0 | 11.0 | 7.5 |
| 4.0 | 10.5 | 13.0 | 9.5 |

The remainder of the feedstuff comprises non-structural carbohydrates, moisture, etc. The first aspect of the invention may further provide a feedstuff comprising a supplemented glutamine source, one or more hydrolysable carbohydrate source and palatability enhancer such as molasses. The feedstuff may optionally provide one or more fibre source, one or more protein source, vitamins, minerals and/or oil. The present invention provides, as a second aspect, the feedstuff according to the first aspect of the invention, for use in increasing or maintaining the level of plasma glutamine in an equine animal. The present invention may be particularly useful for racehorses or other horses that do not receive sufficient to provide a required glutamine content. The need for glutamine in the diet is particularly important during periods of physical stress, such as competitive racing periods.

All preferred features of the first aspect also apply to the second, particularly in respect of levels of glutamine, other components of the feedstuff, composition and nature of the oil etc.

The ability to increase the level of plasma glutamine is particularly useful prior to and during an action or event, which reduces the plasma glutamine level. Such an action or event includes exercise, in particular strenuous exercise such as training or racing, as well as infection, surgery or other stressful or catabolic condition. Levels of glutamine in the feedstuff and quality of feedstuff offered will depend on the particular animal and details of the situation, such as the age of the animal, the health of the animal or the stress suffered or likely to be suffered, amongst others.

It is recognised (see the example) that supplementation of equine feedstuff with glutamine, does not result in accumulative increase in baseline plasma glutamine concentrations. Accordingly, it is important to include the glutamine supplement with the equine feedstuff at a suitable level and at a suitable time interval to provide the increase in plasma glutamine level at the desired time.

Suitable levels of free glutamine or an equivalent (preferably in a supplement) include from 15mg/kg bodyweight, in particular from 30mg/kg bodyweight per day to produce a peak in the plasma glutamine level at 1.5 to 4 hours post-feeding. The feedstuff can provide an increase in the plasma glutamine level above baseline for up to 6 to 8 hours. Accordingly, feeding of such feedstuff every 6 to 8 hours will result in a maintained plasma glutamine level above the baseline. The regularity of feeding will depend on the result to be achieved and on the usual feeding pattern of any particular equine animal. The feeding may be of any pattern (for the present invention) including once, twice or three times daily, optionally with glutamine supplemented snacks and from one, two or three days upwards. The supplemented equine feedstuff may comprise the feedstuff of the animal or can be used as an intermittent feedstuff.

A third aspect of the invention provides a method for increasing or maintaining the level of plasma glutamine in an equine animal, the method comprising the steps of feeding to said equine animal a feedstuff which comprises free glutamine or equivalent level of from 15mg/kg bodyweight, preferably from 30mg/kg bodyweight. Preferably the glutamine level above is supplemented glutamine.

All preferred features of the first and second aspects of the invention also apply to the third aspect, particularly in respect of levels of glutamine, other components of the foodstuff, composition and nature of the oil etc.

The method of the third aspect increases or maintains plasma glutamine levels, providing a pharmacological benefit to said equine animal. Such a pharmacological benefit includes the role of glutamine in the transport of amino nitrogen and ammonia, as a substrate in gluconeogenesis and amoniagenesis, as a final source for rapidly dividing cells, and may also be involved in the regulation of protein synthesis. The pharmacological benefit may also include assisting with the cell-mediated immune response of the animal being fed the supplemented foodstuff.

A fourth aspect of the invention provides the use of a glutamine source, in the manufacture of an equine animal feedstuff, for the prevention or treatment of a pharmacological change associated with a decrease in a plasma glutamine level in an equine animal. The pharmacological change may be in a clinically healthy animal or may be athletically or clinically stressed.

All preferred features of aspects one to three also apply to the fourth, particularly in respect of levels of glutamine, other components of the foodstuff, composition and nature of the oil etc.

The pharmacological change may be associated with one or more of over-training, infection, surgery, other stressful conditions or catabolic stress.

A fifth aspect of the invention provides for the use of a glutamine source as a feedstuff supplement to increase or maintain the plasma glutamine levels in an equine animal.

All preferred features of aspects one to four also apply to the fifth, in particular in respect of levels of glutamine, other components of the foodstuff, composition and nature of the oil, etc.

A sixth aspect of the present invention provides the use of oil and a glutamine in the manufacture of a composition, comprising a fat or oil content of from 4% by weight and a glutamine content of from 15mg/kg bodyweight per day for increasing or maintaining the plasma glutamine level in an equine animal. Increasing or maintaining such plasma glutamine levels is associated with assisting the equine animal in times of athletic or clinical stress. Accordingly, the composition is administered for reducing athletic or clinical stress.

The composition is preferably an equine feedstuff. The equine feedstuff may be as described according to the first aspect of the invention; in the sixth aspect of the invention the glutamine may particularly be provided as supplemented free glutamine or an equivalent thereof (as per the first aspect) or the glutamine may be provided by a food substance (e.g. soya bean meal, linseed, cereal (such as wheat, barley, oats, rye) forages (such as dried grasses), sunflower, lupins, beans, milk powder, copra meal or canola. All other preferred features for the composition of the sixth aspect are as per the foodstuff of the first aspect, including levels of glutamine, other components of a feedstuff, composition and nature of the oil, etc. ,

This sixth aspect of the invention provides an increase in the equine plasma glutamine levels even when the glutamine is provided from a non-supplemented source of free glutamine or an equivalent (i.e. from the raw materials). This advantage is believed to occur due to the combination of the level of oil (or fat) in the composition. As described earlier in this document, oil may have the added benefit of reducing the insulin response to the diet, so enabling further uptake of amino acids and may delay gastric emptying to a more prolonged steady trickle into the gut.

The present invention is described with reference to the figures, in which:
Figure 1 shows mean plasma glutamine concentrations in response to feeding molassed sugar beet (0.5kg), naked oats and mixed feed (both fed according to bodyweight). Hay was fed at 4 hours.
Figure 2 shows mean plasma glutamine response to L-glutamine and glutamine-peptide fed at two doses (95 and 190mg/kg bwt peptide is equivalent to 30 and 60mg/kg bwt L-glutamine content) with molassed sugar beet. Hay was fed at 4 hours.
Figure 3 shows mean changes in plasma ammonia concentrations compared to mean plasma glutamine changes after ingestion of 60mg/kg bwt L-glutamine.
Figure 4 shows mean changes in plasma glutamine concentration when L-glutamine was fed as a single 60mg/kg bwt dose with molassed sugar beet (a.m.) and after 10 days adaptation fed with normal diet at both the a.m. and p.m. feeds. Hay was fed at 4 hours on both occasions.
Figure 5 shows mean plasma glutamine response to commercial mix supplemented with either 60mg/kg bwt L-glutamine or 190mg/kg bwt glutamyl-peptide fed. Hay was fed at 4 hours.
Figure 6a shows glutamine responses to mixed feed only, 60mg/kg bwt L-glutamine with molassed sugar beet, and 60mg/kg bwt L-glutamine with mixed feed. Hay was fed at 4 hours.
Figure 6b shows glutamine responses to commercial mix only, 190mg/kg bwt glutamyl-peptide with molassed sugar beet only, and 190mg/kg bwt glutamyl-peptide with commercial mix. Hay was fed at 4 hours.

The present invention is described with reference to the following example:

### Example

### Summary:

Six clinically healthy horses were offered seven different feeds on different days, on each occasion following an overnight rest. The changes in plasma glutamine were monitored for eight hours post-feeding. The seven feeds were molassed sugar beet (mSB), naked oats (nO), mixed feed (M), and mSB alone or with either 30 or 60mg/kg bwt L-glutamine (G), or the equivalent amount of glutamyl alone or peptide (P). At 1.5h after feeding mean plasma glutamine concentrations were increased by +3% (mSB), +11% (nO) and +43% (M; p<0.05). Feeding mSB supplemented with G, raised mean plasma glutamine levels by +67% (30mg; p<0.05) and +157% (60mg; p<0.05) by 0.75 to 1h. Supplementation with P increased mean plasma glutamine concentrations by +45% (30mg) and +84% (60mg; p<0.05) by 1h. After 10 days of supplementation with 60mg/kg-bwt G, a similar increase (+153%) in concentration was observed (the pre values being unchanged). With the exception of the M, pre levels were regained by 4h in each case. Plasma ammonia and plasma protein concentrations were unaffected by supplementation with glutamine. L-glutamine and P were individually additive to M when fed with M and prolonged the period over which the plasma concentration was increased. Both L-glutamine and P, when added to M, produced an increase of +151% in plasma glutamine with concentrations remaining above pre-values at 8 hours. The effect of glutamine supplementation in the horse has never before been evaluated.

### Materials And Methods

### Horses:

Six stabled Thoroughbred horses (2 mares, 4 geldings; aged 5-9 years), were maintained on hay and mixed feed for a minimum of two weeks before the start of the study. Horses were fed at 8am and 4pm. The mixed feed was a mix of cereal sources, fibre sources and protein sources with added vitamins and minerals. The ingredients included, in descending order of inclusion, enriched fibre pellets, rolled oats, soya hull pellets, flaked maise, flaked barley, flaked peas, molgio, full fat soya, vegetable oils, lucerne (alfalfa), calcium carbonate, dicalcium phosphate, salt, lysine, magnesium phosphate and zinc sulphate. Analysis: oil 8%, protein 12%, fibre 11%, ash 7.5%.

### Experimental Design:

Within each part of the study, the order of the feeds was randomised between horses using a Latin Square design. The study comprised four parts:
*Part 1: Acute plasma glutamine response to a single feed of molassed sugar beet (mSB), naked oats (nO) and mixed feed (M).* The nO and M were fed according to individual bodyweight; mSB was fed as 0.5kg plus 20ml liquid molasses. In all cases 2.5kg hay was offered at 4 hours post feeding; water was provided ad libitum. Total plasma proteins were measured as an indication of plasma volume changes related to the hay or test feeds.
*Part 2: Acute response of mSB supplemented with either L-glutamine (available from Kyowa Hakko, Japan) or glutamyl-peptide (available from DMV International, Veghel, The Netherlands), at doses of either 30 or 60mg*/*kg-bwt in a single feed.* The glutamyl-peptide had an L-glutamine content of 31.5% (reported by the manufacturer) and was fed at 95 and 190mg/kg bwt (equivalent of 30 and 60mg/kg-bwt L-glutamine). The mSB, hay and water were provided as in Part 1.
*Part 3: Chronic response of plasma glutamine over 10 days to base feed supplemented with L-glutamine at a.m. and p.m. feeds.* The L-glutamine was fed at 60mg/kg bwt with the base feed on days 1-9, and with mSB only (as Part 2.) a.m. of day 10 when sampling took place. Hay and water were provided as in Part 1.
*Part 4: Acute response of M supplemented with either L-glutamine or glutamyl-peptide, at a dose of 60mglkg-bwt in a single feed.* The glutamyl-peptide was fed as in part 2 at 190mg/kg bwt (equivalent to 60mg/kg-bwt L-glutamine). The M, hay and water were provided as in Part 1.

### Blood Collection and Sample Storage:

Blood samples were drawn through an indwelling jugular catheter into tubes containing lithium heparin (on ice). In Part 1 samples were taken pre and at 0.5, 1, 1.5, 2, 3, 4, 6 and 8 hours post feed. In Parts 2, 3 and 4 samples were taken pre and at 0.25, 0.5, 0.75, 1, 1.5, 2, 3, 4, 6 and 8 hours. Samples were centrifuged, the plasma harvested, divided into aliquots and stored at -80°C.

### Analysis:

Plasma was deproteinised with an equal volume of perchloric acid and neutralised with KHCO₃. Glutamine was assayed by conversion to glutamate and the formation of formazan dye. L-Asparaginase (EC3.5.1.1; 5mg/ml from *Escherichia coli* (from Boehringer Mannheim/Roche, Lewes, East Sussex, England) hydrolyses glutamine to glutamate producing ammonia, addition of glutamate dehydrogenase (EC1.4.1.2; 10mg/ml from bovine liver) converts glutamate to 2-oxoglutarate, NADH and ammonium. In the presence of diaphorase (EC1.8.1.4; from *Clostridia kluvyeri* 300U/ml, from ICN Biomedicals, Theme, Oxfordshire, England) INT (Aldrich Chemical Co., Gillingham, Dorset, England) oxidises NADH producing a pink formazan dye. Colour development after 30 minutes incubation in the dark at 20°C, was read photometrically at 492nm on a microplate reader (SPECTRAmax PRO 250 with SOFTmax PRO software vs. 1.2.0 for Apple Mac, 1994, Molecular Devices Corporation, California 94089, USA). The method was a consolidation of those by Lund (1974), and, Beutler and Michal (1974), creating a one-step enzymic method applied to a microplate reader using only 20µl of sample.

Each sample was assayed as a 'test' and a 'blank'. L-Asparaginase was added to the 'test' reagent (for the conversion of L-glutamine to L-glutamate), but was replaced with water in the 'blank' reagent in order to measure the background L-glutamate content of the sample. The L-glutamine concentrations were calculated from the absorbance change between test and blank. Samples were run in duplicate, and each group of assays was controlled by simultaneously assaying a series of known standards and QC plasma extracts.

Plasma ammonia was determined in samples from Part 2 where the higher dose of L-glutamine (60mg/kg-bwt) was fed, using a dry-strip analyser (Ammonia Checker II, Kyota Daiicha Kagaku Co Ltd., Japan) pre-validated for equine plasma. Total plasma protein content was estimated using a refractometer (Goldberg Refractometer).

### Statistical Analysis:

Where appropriate, the results were tested for significance using paired students t-tests and ANOVA for repeated measures. P<0.05 was taken as indicating a significant difference. All results are given as means ±SD. Areas under the plasma concentration-time curve (AUC), 0-4h and 4-8h post each treatment were calculated using the trapezoidal rule (see table 5).

### Results

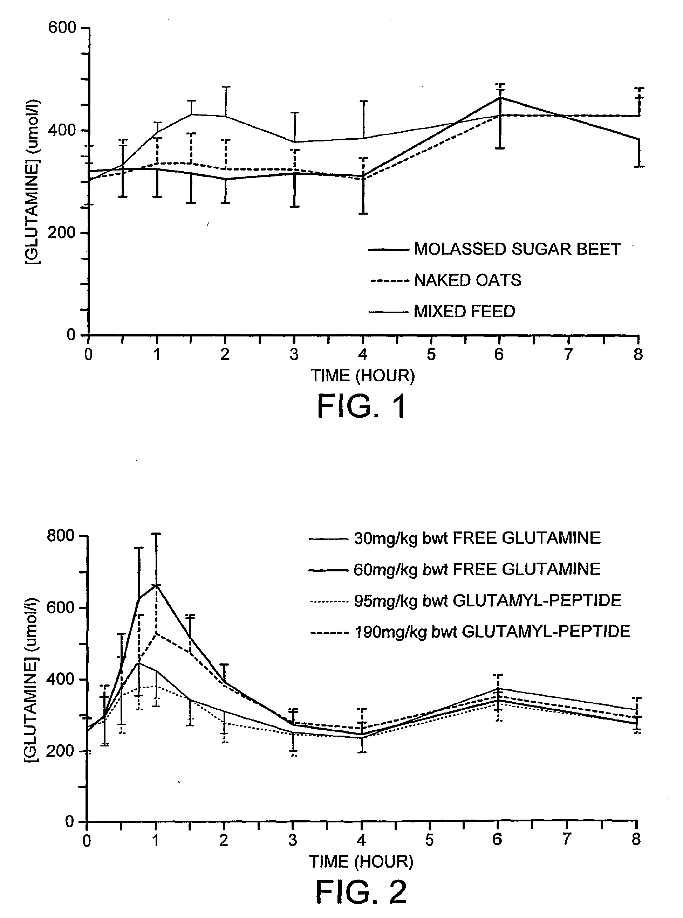

### Part One (figure 1; table 1)

Ingestion of molassed sugar beet had no effect on the plasma glutamine concentration. The naked oats produced an average change of +11% in plasma glutamine from a mean of 301.6 (±65.1) to 336.1 (±55.6) µmol/l by 1.5h post feeding. The mixed feed produced a significant increase (p<0.05) of +43% by 1.5h. The glutamine concentrations declined to pre values over the following 1.5 hours with the oats and sugar beet but remained significantly (p<0.05) elevated above baseline concentration with the mix. The mix kept the levels elevated until at least four hours post feeding.

Hay fed at 4h produced a mean increase in glutamine concentration (µmol/l) by 6h post of: +157.1 (mSB; p<0.05), +134.4 (nO; p<0.05) and +43.9 (CM) from the minimum values observed at 4h. The absolute concentrations of plasma glutamine at the 6h peak were comparable between food types: mSB 465.5 (±102.3), nO 431.9 (±52.8) and CM 430.6 (±35.3) µmol/l.

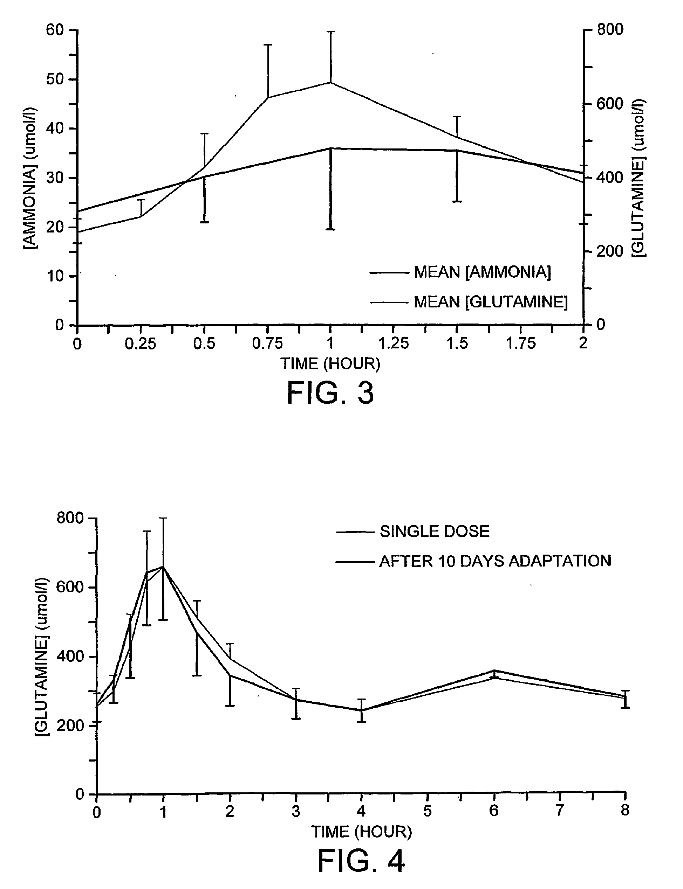

### Part Two (figures 2 & 3; tables 2 & 3)

At 30mg/kg bwt L-glutamine (30mgG) produced a peak at 0.75 hours post of +67% from the pre feed value. The peaks in all other supplement doses were observed at 1h post: +45%, +157% and +84%, for the 95mg/kg bwt glutamyl-peptide (95mgP), 60mg/kg bwt free glutamine (60mgG), and the 190mg/kg bwt glutamyl-peptide (190mgP) dose, respectively.

Hay fed at 4h post produced a mean increase in glutamine concentration by 6h of +132.3, +94.5, +86.7 and +84.8 µmol/l (30mgG, 95mgP; 60mgG and 190mgP respectively) from the nadir in values observed at 4 hours is a likely result of fluid shifts in GI tract in response for hay feeding. The absolute plasma glutamine concentrations at the 6h peak were comparable between supplement types and doses: 30mgG 363.9 (±54.7), 95mgP 323.8 (±43.0), 60mgG 333.2 (±23.2) and 190mgP 347.7 (±57.3). There was no significant increase in ammonia observed with the administration of 60mgG (table 3; figure 4).

### Part Three (figures 4; table 4)

No change was observed in baseline glutamine concentrations after 10 days supplementation. The results show the plasma glutamine response to 60mg/kg-bwt L-glutamine to be highly reproducible when compared with the initial dose given on the first day (increases of +157 and +153% respectively; figure 3).

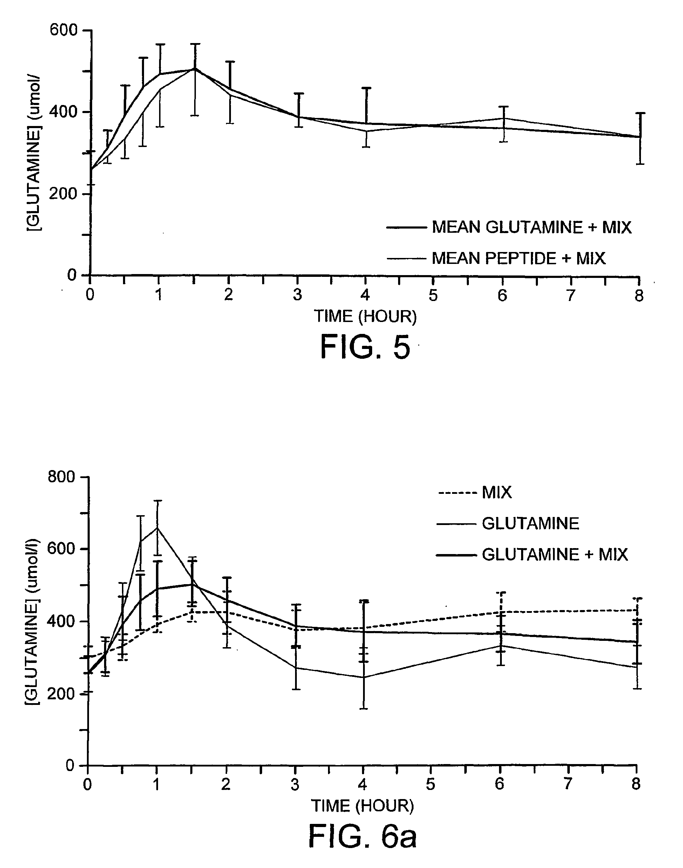

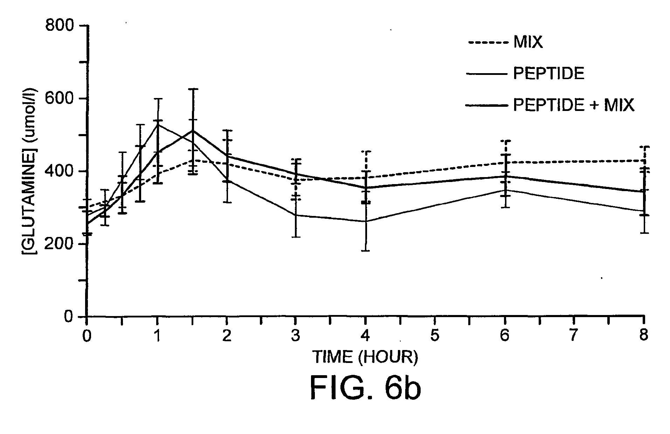

### Part Four (figures 5 & 6a/b; table 5)

The supplementation of M with either 60mgG or 190mgP produced a significant increase (p<0.05) of +50% in plasma glutamine concentration at 1.5h post. Mean plasma glutamine concentrations increased by +246.3 and +251.9 µmol/l when fed M + G and M + P respectively.

Hay fed at 4h resulted in an 8% increase only in the glutamine concentration during the feeding of M + G (p>0.05).

### Total Plasma Protein (TPP) Results:

Significant changes in TPP were observed only between 4 and 6h when hay was fed after naked oats (p<0.05), and at 1.5h after feeding the mix (p = 0.05). Results ranged from 5.5-6.1g/dl, which are within the normal resting range reported for horses (5.5-7.5g/dl; Rose & Hodgson (1994), Haematology and Biochemistry. In: The Athletic Horse: Principles and Practice of Equine Sports Medicine. Eds. D.R. Hodgson & R.J. Rose).

### Discussion

In Part one, feeding of naked oats and compound mix elicited a protracted increase in plasma glutamine concentration at 1.5 hours, which had returned to baseline levels by four hours. In the latter study, a decline in amino acid concentrations was evident following the peak this being attributed, in part, to the action of insulin. Insulin promotes the uptake of amino acids by muscle and other tissues, and consequently, plasma amino acid concentrations may decrease below pre-feeding levels during the post absorptive period. A decline below baseline was seen also in plasma glutamine in the present study.

Molassed sugar beet did not produce any change in plasma glutamine concentration, presumably due to its minimal protein content. The 11% increase in concentration induced by the oats was, however, surprisingly small when compared to the 43 % increase observed in the mix which had a comparable protein content (12%). This response may reflect differences in protein structure, availability, and/or the actual glutamine content of oats and the higher oil content of the mix (as discussed above). Again this may reflect that the proteins in cereal grains are generally of poor nutritional value.

In Part two, L-glutamine produced a greater response (peak concentration and area under the plasma concentration-time curve [AUC]) than the peptide, suggesting that glutamine fed in the free form as an oral bolus is capable of reaching the equine circulation in greater quantities than glutamine fed as a peptide (table 5; figure 2). Free glutamine in powder form is stable, but degrades to glutamate and ammonia in solution, particularly at low pH and/or with increased temperatures, for instance during feed manufacture. Still higher doses per kg bodyweight of the glutamyl-peptide could potentially yield results comparable to the 60mg/kg-bwt free-glutamine observed here.

There was no effect of a ten-day period of adaptation to glutamine, supplementation in the healthy horse, neither increasing nor decreasing the plasma response. Neither did oral ingestion of glutamine have any effect on plasma ammonia concentrations. The highest plasma ammonia value recorded in this study was 64µmol/l at 1h following ingestion of 60mg/kg bwt glutamine. Resting ammonia concentrations can increase to as high as 800 (±397) µmol/l within 15 minutes of treadmill exercise with no apparent detrimental effects to the horse. The small increases observed here therefore suggest a minimum risk of clinically significant hyperammonaemia with glutamine doses up to 60mg/kg bwt.

The mean increase in equine plasma glutamine concentration of 157% at 1h post feeding in response to the 60mg/kg-bwt dose of L-glutamine was highly significant (p<0.01). The observed increase, however, represents just 8% of the total glutamine ingested assuming a plasma volume of 40 litres. The return to baseline within three hours of the maximum concentration suggests a rapid removal from the circulation through utilisation or excretion.

In Part four, when L-glutamine and glutamyl-peptide were fed in combination with the mixed feed, the responses observed were comparable. These two supplements were fed at doses equal to or equivalent to 60mg/kg bwt, and there was an additional increase in plasma glutamine of +48% above that produced by the mix alone (see Table 5). Furthermore, glutamine was present in the plasma for a longer period than when either supplement was provided with the molassed sugar beet.

Previous studies in the horse have shown the healthy equine intestine to be an avid utiliser of glutamine in vivo (Duckworth D.H., Madison, J.B., Calderwood-Mays, M. & Souba, W.W. (1992) Arteriovenous differences for glutamine in the equine gastrointestinal tract. Am. J. Vet. Res., 53, 1864-1867). The horses in this study were clinically healthy and the response recorded here reflects glutamine transport by the healthy equine intestine. Whilst there may still be a substantial metabolism of the glutamine by the enterocytes, it is potentially not of the same order as in a damaged or malfunctioning gut.

It can be assumed, therefore, that the response elicited here was in direct response to the test feeds and supplements and not the result of other feeding or gut-related factors. It is unlikely that the over night fast prior to sampling during this study will not have influenced the response. In the horse, it is known that starvation and fasting in excess of 24 hours can promote amino acid mobilisation from tissues into, and faster removal from, the circulation. Brush-border transport of glutamine into, and its subsequent metabolism by enterocytes is also decreased during starvation, thereby contributing to the increased transport of the limited available glutamine into plasma.

Naked oats used in the example (standard) have a content of approximately 18g/kg oats as fed. Taking the various horse bodyweights and the amounts fed into consideration, this results in approximately 36mg/kg bodyweight glutamine (per day). Despite the glutamine content (and the fact that oat starch is very available), little plasma glutamine rise was noted. Molassed sugar beet pulp used contains approximately 17g glutamine per kg sugar beet, so 0.5kg sugar beet contains around 8.5g glutamine per 475 kg horse i.e. approximately 18mg/kg bodyweight, which showed no plasma glutamine increase at all.

The present study has demonstrated that glutamine, supplied in various forms, may be taken up and, in the short term, increase circulating plasma glutamine concentrations in equine animals.

**Table 1: mean (±SD) plasma glutamine concentrations (µmol/l) for each feed presenting pre, peak and lowest value (nadir).**

| Feed | Pre | Peak | Nadir |
|---|---|---|---|
| Sugar beet | 314.8 (60.9) | 324.7 (53.0) | 308.4 (71.1) |
| Naked oats | 301.6 (65.1) | 336.1 (55.6) | 297.6 (47.2) |
| Mixed feed | 298.7 (33.5) | 428.3 (26.9) | 378.2 (53.4) |

**Table 2: mean (±SD) increase of plasma glutamine concentrations (µmol/l) in response to feeding L-glutamine (G) and glutamyl-peptide (P) at each dose (P is shown as actual mg/kg-bwt fed).**

| Supplement | Pre | Peak | Nadir |
|---|---|---|---|
| 30mg/kg G | 264.9 (62.3) | 442.8 (93.2) | 231.6 (36.8) |
| 95mg/kg P | 263.1 (69.9) | 380.3 (36.3) | 229.3 (30.2) |
| 60mg/kg G | 256.5 (35.7) | 660.2 (139.6) | 246.5 (29.9) |
| 190mg/kg P | 284.7 (79.9) | 524.7 (133.3) | 262.9 (49.4) |

**Table 3: mean (±SD) changes in plasma ammonia concentration (µmol/l) in response to feeding molassed sugar beet supplemented with L-glutamine at 60mg/kg-bwt. Results recorded at times representing the period of peak and decline in plasma glutamine concentrations.**

| Time (hour) | Mean | SD | Range |
|---|---|---|---|
| 0 | 23.5 | *6.6* | 15-31 |
| 0.5 | 30.2 | *9.1* | 20-45 |
| 1 | 36.0 | *16.3* | 22-64 |
| 1.5 | 35.8 | *10.3* | 18-44 |
| 2 | 31.4 | *10.5* | 21-46 |

**Table 4: mean (±SD) increase of plasma glutamine concentrations (µmol/l) in response to feeding commercial mix (CM) supplemented with 60mg/kg bwt L-glutamine (G) or 190mg/kg bwt glutamyl-peptide (P).**

| Feed | Pre | Peak | Nadir |
|---|---|---|---|
| 60mg/kg G+M | 257.9 (47.9) | 504.2 (63.0) | 344.1 (59.7) |
| 190mg/kg P+M | 257.5 (32.1) | 509.8 (118.9) | 357.2 (41.5) |

**Table 5: mean (±SD) areas under the curve (µmol/l of glutamine/30 minutes) calculated for each part of the study. Hay was fed at 4h.**

| Feed | Pre to 4h | |
|---|---|---|
| | Area | *SD* |
| Sugar beet | 124 | *54* |
| Naked oats | 130 | *58* |
| Mixed feed | 344 | *122* |
| 30mg/kg G | 313 | *121* |
| 30mg/kg P | 299 | *105* |
| 60mg/kg G | 595 | *92* |
| 60mg/kg P | 495 | *121* |
| 10 day adaptation to 60mg/kg G | 583 | *216* |
| 60mg/kg G + M | 648 | *115* |
| 60mg/kg P+M | 586 | *94* |

## Claims

1. Use of a glutamine source in the manufacture of an equine animal feedstuff, to increase or maintain the plasma glutamine levels in an equine animal, wherein the equine animal feedstuff comprises a level of free glutamine of from 15mg/kg to 200mg/kg body weight per day or an equivalent level from an alternative glutamine source.

2. Use, as claimed in claim 1, wherein increasing or maintaining the plasma glutamine levels prevents or treats a pharmacological change reflected by a decrease in plasma glutamine levels in an equine animal wherein the pharmacological change is associated with one or more of over-training, infection, surgery, other stressful conditions or catabolic stress.

3. Use, as claimed in claim 1, wherein increasing or maintaining the plasma glutamine levels assists with the cell mediated immune response of the animal.

4. The use claimed in any one of claims 1 to 3, wherein the glutamine source is free amino acid L-glutamine or a peptide rich in L-glutamine.

5. The use as claimed in any one of claims 1 to 4, wherein the peptide rich in L-glutamine is a dipeptide.

6. The use as claimed in any one of claims 1 to 5 which comprises one or more of a hydrolysable carbohydrate source, a fibre source and a protein source.

7. The use, as claimed in any one of claims 1 to 6, which is in the form of a snack bar or a drink.

8. Use as claimed in claim 1, further comprising the use of an oil, wherein the oil content of the equine animal feedstuff comprises from 4% by weight.

9. Use, as claimed in claim 8, wherein the glutamine source is one or more of oats, soya bean meal, linseed, cereals, forages, sunflower, lupins, beans, milk powder, copra meal or canola.

10. Use, as claimed in claim 8, wherein the free-glutamine or equivalent level is of from 50mg/kg bodyweight per day.

11. Use, as claimed in claim 8, wherein the feedstuff further comprises 4% to 15% by weight fat or oil.

## Patentansprüche

1. Verwendung einer Glutaminquelle bei der Herstellung eines Futtermittels für pferdeartige Tiere, um die Plasmaglutaminniveaus bei einem pferdeartigen Tier zu erhöhen oder zu erhalten, wobei das Futtermittel für pferdeartige Tiere einen Gehalt an freiem Glutamin von 15 mg/kg bis 200 mg/kg Körpergewicht pro Tag oder einen Äquivalentgehalt aus einer alternativen Glutaminquelle umfasst.

2. Verwendung nach Anspruch 1, wobei das Erhöhen oder Erhalten des Plasmaglutaminniveaus eine pharmakologische Änderung verhindert oder behandelt, die widergespiegelt wird durch eine Abnahme der Plasmaglutaminniveaus bei einem pferdeartigen Tier, wobei die pharmakologische Änderung assoziiert ist mit einem oder mehreren eines Übertrainierens, einer Infektion, einer Operation, anderen stressigen Zuständen oder katabolischem Stress.

3. Verwendung nach Anspruch 1, wobei das Erhöhen oder Erhalten der Plasmaglutaminniveaus die zell-vermittelte Immunreaktion des Tieres unterstützt.

4. Verwendung nach einem der Ansprüche 1 bis 3, wobei die Glutaminquelle freie Aminosäure L-Glutamin oder ein an L-Glutamin reiches Peptid ist.

5. Verwendung nach einem der Ansprüche 1 bis 4, wobei das an L-Glutamin reiche Peptid ein Dipeptid ist.

6. Verwendung nach einem der Ansprüche 1 bis 5, welche eine oder mehrere einer hydrolysierbaren Kohlenhydratquelle, einer Faserquelle und einer Proteinquelle umfasst.

7. Verwendung nach einem der Ansprüche 1 bis 6, welche in der Form eines Snack-Riegels oder eines Getränks ist.

8. Verwendung nach Anspruch 1, weiter umfassend die Verwendung eines Öls, wobei der Ölgehalt des Futtermittels für pferdeartige Tiere ab 4 Gew.-% umfasst.

9. Verwendung nach Anspruch 8, wobei die Glutaminquelle eine oder mehrere von Hafern, Sojabohnenmehl, Leinsamen, Cerealien, Grünfutter, Sonnenblumen, Lupinen, Bohnen, Milchpulver, Copramehl oder Raps ist.

10. Verwendung nach Anspruch 8, wobei der Gehalt an freiem Glutamin oder an Äquivalent von 50 mg/kg Körpergewicht pro Tag ist.

11. Verwendung nach Anspruch 8, wobei das Futtermittel ferner 4% bis 15 Gew.-% Fett oder Öl umfasst.

## Revendications

1. Utilisation d'une source de glutamine dans la fabrication d'un aliment pour équidés, permettant d'augmenter ou de maintenir les taux de glutamine plasmatique chez un équidé, dans laquelle l'aliment pour équidés comprend un taux de glutamine libre allant de 15mg/kg à 200mg/kg de poids corporel par jour ou un taux équivalent provenant d'une source alternative de glutamine.

2. Utilisation, selon la revendication 1, dans laquelle l'augmentation ou le maintien des taux de glutamine plasmatique permet de prévenir ou de traiter une modification pharmacologique revélée par une diminution des taux de glutamine plasmatique chez un équidé, dans laquelle la modification pharmacologique est associée à une ou plusieurs des conditions suivantes : sur-entraînement, infection, chirurgie, autres conditions stressantes ou stress catabolique.

3. Utilisation, selon la revendication 1, dans laquelle l'augmentation ou le maintien des taux de glutamine plasmatique contribue à la réponse immune médiée par les cellules de l'animal.

4. Utilisation selon l'une quelconque des revendications 1 à 3, dans laquelle la source de glutamine est l'acide aminé libre L-glutamine ou un peptide riche en L-glutamine.

5. Utilisation selon l'une quelconque des revendications 1 à 4, dans laquelle le peptide riche en L-glutamine est un dipeptide.

6. Utilisation selon l'une quelconque des revendications 1 à 5, qui comprend une ou plusieurs sources parmi un carbohydrate hydrolysable, une source de fibres et une source de protéines.

7. Utilisation, selon l'une quelconque des revendications 1 à 6, qui est sous la forme d'une barre de collation (snack bar) ou d'une boisson.

8. Utilisation selon la revendication 1, comprenant en outre l'utilisation d'une huile, dans laquelle la teneur en huile de l'aliment pour équidés est d'au moins 4% en poids.

9. Utilisation, selon la revendication 8, dans laquelle la source de glutamine est choisie parmi un ou plusieurs des aliments suivants : avoines, farine de soja, graines de lin, céréales, fourrages, tournesol, lupins, haricots, poudre de lait, farine de copra ou colza.

10. Utilisation, selon la revendication 8, dans laquelle le taux de glutamine libre ou un équivalent est d'au moins 50mg/kg de poids corporel par jour.

11. Utilisation, selon la revendication 8, dans laquelle l'aliment comprend en outre 4% à 15% en poids de matières grasses ou d'huile.
